Europäisches Patentamt

European Patent Office

Office européen des brevets

(19)

(11) Veröffentlichungsnummer: **0 592 484 B1**

(12) # EUROPÄISCHE PATENTSCHRIFT

(45) Veröffentlichungstag der Patentschrift: **18.01.95**

(51) Int. Cl.⁶: **A61K 9/00**, A61K 9/46

(21) Anmeldenummer: **92912980.7**

(22) Anmeldetag: **24.06.92**

(86) Internationale Anmeldenummer:
**PCT/EP92/01421**

(87) Internationale Veröffentlichungsnummer:
**WO 93/00886 (21.01.93 93/03)**

(54) **REAKTIONSDOTIERTE BRAUSESYSTEME.**

(30) Priorität: **01.07.91 CH 1942/91**

(43) Veröffentlichungstag der Anmeldung:
**20.04.94 Patentblatt 94/16**

(45) Bekanntmachung des Hinweises auf die
Patenterteilung:
**18.01.95 Patentblatt 95/03**

(84) Benannte Vertragsstaaten:
**AT BE CH DE DK ES FR GB GR IT LI NL SE**

(56) Entgegenhaltungen:
**EP-A- 0 233 853       WO-A-88/00009**
**WO-A-91/07174         AT-A- 374 348**
**US-A- 3 887 700        US-A- 4 678 661**

**DATABASE WPIL Section Ch, Week 8219,**
**Derwent Publications Ltd., London, GB;**
**Class A06, AN 82-38452E & JP,A,57056434**

(73) Patentinhaber: **Gergely, Gerhard, Dr.**
**Gartengasse 8,**
**Postfach 153**
**A-1053 Wien (AT)**

(72) Erfinder: **GERGELY, Gerhard**
**Gartengasse 8,**
**Postfach 153**
**A-1053 Wien (AT)**
Erfinder: **GERGELY, Thomas**
**Gartengasse 8,**
**Postfach 153**
**A-1053 Wien (AT)**
Erfinder: **GERGELY, Irmgard**
**Gartengasse 8,**
**Postfach 153**
**A-1053 Wien (AT)**

(74) Vertreter: **Büchel, Kurt F., Dr.**
**Patentbüro Dr. Büchel**
**Letzanaweg 25**
**FL-9495 Triesen (LI)**

EP 0 592 484 B1

EP 0 592 484 B1

**Beschreibung**

Die Erfindung betrifft eine Brausetablette nach dem Oberbegriff des Anspruchs 1, wie sie zum Beispiel nach dem Verfahren gemäss WO 88/00009 hergestellt werden kann. Zusammensetzung und Herstellung bisher bekannter Brausesysteme beruhen auf der Mischung von organischen, essbaren Säuren mit Kohlensäure abspaltenden Alkalien und/oder Erdalkalien, die gemeinsam granuliert oder gemeinsam zur Reaktion gebracht, getrocknet und mit Wirkstoffen verpresst werden. Bei diesen Verfahren liegen sämtliche Substanzen in mehr oder weniger pulverisierter oder feinkristalliner Form vor, wobei die Eigenschaften des entstehenden Produktes dem statistischen Querschnitt der Mischungspartner entsprechen.

Bei dem eingangs genannten bekannten Brausesystem weist der Trägerkristall im Inneren einen reaktiven Kern aus z.B. Zitronensäure, im Äusseren aber Mononatriumcitrat auf, wie es durch die individuelle Reaktion ad hoc gebildet wurde. Somit hat das Partikel aussen die chemischen Eigenschaften des Mononatriumcitrats; im Innern aber besteht es nach wie vor aus Zitronensäure. Wenn also bei Zutritt von Wasser die erste Schicht Mononatriumcitrat weggelöst ist, kommt es zur stürmischen Reaktion mit der innerhalb des Kernes liegenden, nativen Zitronensäure.

Aus den verschiedensten Gründen ist man bestrebt, pharmazeutische Zubereitungen in Form von Brausetabletten auf den Markt zu bringen: Einerseits haben viele Patienten Schluckschwierigkeiten, besonders bei grossen Tabletten; andererseits sollen viele Wirkstoffe mit wenigstens einer wenn auch bescheidenen Wassermenge eingenommen werden, damit sie nicht konzentriert auf die Magenschleimhaut treffen.

Nun hatte man bisher zwei Probleme mit Brausetabletten:

Einerseits gibt es viele Wirkstoffe, die selbst feuchtigkeitsempfindlich sind, bzw. bei Vorliegen von Feuchtigkeit mit einem der Bestandteile des Brausesystems reagieren und dadurch schlecht lagerfähig sind, insbesondere auch weil die meist verwendete Zitronensäure so gut wie immer eine wenn auch geringe Restfeuchtigkeit von 0,2-0,4% enthält. Man war also bisher meist auf sehr teure, feuchtigkeitsdichte Verpackungen angewiesen.

Auch sehr leicht lösliche Wirkstoffe bieten Probleme, weil sie beim beginnenden Auflösen in Wasser rasch eine konzentrierte Lösung bilden und dadurch die Reaktion der Brausebestandteile miteinander behindern bzw. verlangsamen.

Andererseits sind bisher, besonders für grosse Wirkstoffgaben von z.B. 1 g pro Dosis, zur Erzielung annehmbarer Auflösungszeiten sehr grosse Brausetabletten von meist 3-4 g Gesamtgewicht erforderlich, die wiederum grosse Flüssigkeitsmengen zur Auflösung benötigen; solche grosse Flüssigkeitsmengen werden aber von vielen Patienten auch nicht gerne eingenommen. Darüber hinaus sind sie sowohl vom Materialaufwand wie auch von der Verpackung her teuer und führen dem Körper des Patienten gegebenenfalls grosse Mengen unerwünschter Natriumionen zu. Schliesslich haben grosse Brausetabletten oft eine für bestimmte Anwendungsfälle unerwünscht hohe Pufferkapazität bzw. Säurebindungsvermögen.

Eine weitere Schwierigkeit ergibt sich aus der Notwendigkeit, Bindemittel einzusetzen, um die Brausemischungen überhaupt zu stabilen, genügend harten Tabletten verpressen zu können. Solche Bindemittel, wie beispielsweise Sorbitol, Polyvinylpyrrolidon u.a. vergrössern zwar die Härte von Brausetabletten, verlangsamen aber deutlich den Zerfall und machen die Tabletten noch zusätzlich feuchtigkeitsempfindlich.

Nun hat man bereits versucht, die Lagerstabilität empfindlicher Wirkstoffe in Brausetabletten zu erhöhen, indem man als gasabspaltenden Bestandteil z.B. wasserfreies Kaliumcarbonat (US-PS 3,136,692) oder als sauren Bestandteil Mononatriumcitrat (DE-A1 3920626 oder US-PS 4,689,218) eingesetzt hat; solche Tabletten brauchen dann aber sehr lange zum Auflösen, weil Kaliumcarbonat selbst mit Zitronensäure viel langsamer reagiert als beispielsweise Natriumbicarbonat, bzw. letzteres mit Mononatriumcitrat sehr viel langsamer reagiert als mit Zitronensäure.

Für leicht lösliche Wirkstoffe hatte man auch bereits eine Kombination von Zerfallsmitteln mit dem Brausesystem vorgeschlagen (WO 91/07174), weil eine beim Erstkontakt der Tablette mit Wasser ohne Zerfallsmittel zunächst entstehende, konzentrierte Wirkstofflösung die Reaktion der Brausebestandteile miteinander hindert. Dies ermöglicht auch die Herstellung kleinerer Gesamttablettengewichte; leider ergibt sich hier jedoch wiederum der unerwünschte Effekt, dass die Lösungen durch das Zerfallsmittel trüb werden.

Nun hatte man in der eingangs genannten WO 88/00009 bereits vorgeschlagen, die Lagerstabilität und Reaktionsfähigkeit einer Brausetablette dadurch zu verbessern, dass man eine geringfügige Anreaktion der sauren und der carbonatischen Bestandteile des Brausesystems herbeiführte, wobei sich - wie erwähnt - eine Brausetablette nach dem Oberbegriff des Anspruches 1 ergibt. Es hat sich jedoch gezeigt, dass man damit die soeben geschilderten Probleme nur sehr unzureichend löst.

Die Erfindung hat sich daher die Aufgabe gestellt, eine Brausetablette zu schaffen, die auch mit feuchtigkeitsempfindlichen und/oder mit wenigstens einem der Bestandteile des Brausesystems zur Reaktion neigenden und/oder leicht löslichen, pharmazeutischen Wirkstoffen eine gute Lagerstabilität aufweist, bzw. die Herstellung kleinerer Brausetabletten, z.B. von nur 1,5 bis 2 Gramm für bis zu 1 Gramm pharmazeutischen Wirkstoffes, ermöglicht, die sich in möglichst kurzer Zeit auflösen, wobei die resultierende Lösung möglichst klar sein soll. Schliesslich soll das System auch ohne Verwendung von auflösungsverzögernden Bindemitteln leicht verpressbar sein und für bestimmte Anwendungsfälle Brausetabletten von möglichst geringem Säurebindungsvermögen zu schaffen.

Eine zufriedenstellende Lösung dieser Aufgaben ergibt sich in überraschender Weise erfindungsgemäss durch die im Kennzeichen des Anspruches 1 beschriebenen Massnahmen. Weitere und verbesserte Ausführungsformen der Erfindung sind in den Kennzeichen der abhängigen Ansprüche beschrieben.

Die vorliegende Erfindung beruht auf der Herstellung von Brausepartikeln mit individueller Struktur, wobei die inneren und äusseren Eigenschaften der Brausepartikel mittels verschiedener neuartiger Massnahmen quasi programmiert werden können.

Grundvoraussetzung für diese neue Technologie ist die Verwendung von Trägerkristallen aus essbaren, organischen Säuren, weil diese regulär kristallisieren, in geeigneten Kristallgrössen zur Verfügung stehen und durch ihre physikalischen Eigenschaften zur Dotierung besonders geeignet sind. Je leichter löslich eine Säure ist, desto besser können ihre Kristalle benetzt und anschliessend abgedeckt bzw. passiviert werden. Allerdings muss die Säure jeweils im Überschuss und in geeigneter Korngrösse vorliegen, damit wirklich nur die Oberfläche benetzt wird und nicht womöglich eine teigige Masse entsteht, die nicht mehr erfindungsgemäss weiterverarbeitet werden könnte.

Carbonate und Bicarbonate scheiden als Trägerkristalle aus, weil sie sich nicht dotieren lassen; sie weisen Wasser ab.

Der einfachste Fall liegt vor, wenn man definierte Zitronensäurekristalle, etwa in der Grössenordnung von 0,1 bis 0,6 mm, insbesondere 0,2 bis 0,4 mm Kristallgrösse, mit Wasser, oder einer Pufferlösung (WO 88/00009), oder einer konzentrierten Gluconsäure-delta-lacton-Lösung (US-A-4678661, Beispiel 4) benetzt, mit Erdalkalicarbonaten reagieren lässt und diese Reaktion zu einem gewissen Zeitpunkt abstoppt. Danach entstehen individuelle Brausepartikel, die bereits gegen Feuchtigkeit relativ beständig sind, aber nicht für sämtliche Wirkstoffe und nicht für sämtliche möglichen Variationen geeignet sind. Treibt man nämlich die Reaktion zu weit, d.h. wandelt man etwas mehr als 5 oder 10% der Säureoberfläche in Alkalisalze um, dann wird das Brausesystem zu langsam.

In Beispiel 4 der erwähnten US-A-4678661 handelt es sich dabei um eine andere Aufgabenstellung (es soll eine natriumarme - und nicht eine besonders kleine! - Brausetablette hergestellt werden), um eine andere technische Massnahme zur Lösung dieser Aufgabe (als erste Schicht eines gasabspaltenden Bestandteils wird immer Calciumcarbonat aufgetragen, was in der gegenständlichen Erfindung fehlt) und um ein anderes Ergebnis (es werden Standard-Brausetabletten von 3.5 bzw. 4 g - und nicht wie erfindungsgemäss von etwa 2 Gramm oder sogar noch weniger - hergestellt).

Erfindungsgemäss wurde nun gefunden, dass das System in der Reaktion beschleunigt werden kann, wenn man gleichzeitig in die Oberfläche Fremdsäuren einbaut. So werden z.B. in mehreren Schichten Alkalisalze mehrerer Säuren gebildet, wobei vorzugsweise 10 bis 40 % des Brausesystems zum Salz - insbesondere nur zum Monosalz - durchreagiert sind.

Schliesslich gestattet die Erfindung durch die Möglichkeit der Herstellung kleiner Brausetabletten auch den Gehalt an Natriumonen gering zu halten und Brausetabletten mit nur sehr geringem Säurebindungsvermögen (acid consuming capacity) von z.B. $\leq$ 5 meq herzustellen.

Man kann also beispielsweise in eine Zitronensäure-Kristalloberfläche, vorzugsweise auf Temperaturen zwischen 50 und 60 $^\circ$C erwärmt, mittels insbesondere sehr konzentrierter, wässeriger, alkoholischer bzw. wässerig-alkoholischer Lösungen Äpfelsäure, Weinsäure oder auch Gluconsäure-delta-lacton einbauen und diese fremdsäuredotierte Kristalloberfläche hernach mit Alkalicarbonaten und/oder -bicarbonaten und gegebenenfalls - insbesondere im Falle von Gluconsäure-delta-lacton - mit weiteren Säuren unterschiedlichen pK-Wertes reagieren lassen.

Es kommt übrigens auf die physikalischen und chemischen Eigenschaften der zu verarbeitenden Wirkstoffe an, wie man diese Systeme herstellt, ob man z.B. Äpfelsäure und Zitronensäure - beide als Trägerkristall - verwendet und darauf dann entweder eine Zitronensäurelösung oder Gluconsäure-delta-lacton-Lösungaufbringt und anschliessend das Bicarbonat anreagieren lässt. Immer aber bleibt die Dotierung mit einer Fremdsäure, bzw. mit dem Gluconsäure-delta-lacton, und auch die Umsetzung in das Monosalz. Die Verhältnisse der Säuren zueinander bewegen sich im Normalfall, z.B. bei Zitronensäure zu Äpfelsäure, um 8:1, können aber auch 3:1 betragen.

Solche Systeme lösen sich wesentlich schneller auf, weil durch die verschiedenen pH- und pK-Werte der zwei oder mehreren Säuren das Entstehen einer reaktionsbremsenden Pufferlösung während des Auflösens lokal ständig gestört und dadurch verhindert wird. Bei den erfindungsgemässen Brausepartikeln liegt die solches bewirkende Gitterstörung bereits vor und muss nicht erst entstehen, wie es zum Beispiel bei bisher schon gelegentlich gehandhabten Mischungen zweier verschiedener Säuren der Fall wäre.

Zum Beispiel stellt sich an der Grenzschicht eines Zitronensäure-Kristalles ein Gleichgewichts-pK von 3,14 ein; liegt dort nun aber eine andere Säure, mit höherem oder tieferem pK-Wert im Gitter vor, wird das Gleichgewicht gestört; es bildet sich kein stabiler Puffer, und die Einzelkörner - und damit die Tablette - lösen sich schneller auf. Es müssen aber beide Säuren partiell anreagiert sein; ist dies nur für eine Säure der Fall, dann ist die Tablette allenfalls nicht ausreichend lagerstabil.

Bei einer dreibasischen, mittelschwachen Säure hängt der pH-Wert nach einer Gleichung vierten Grades vom pK-Wert ab; es bilden sich dann an verschiedenen Stellen verschiedene pH-Werte bzw. Pufferungen aus.

Das System lässt sich nochmals verändern, wenn man beispielsweise Wirkstoffe einbauen möchte, die gegen Alkalien empfindlich sind. In diesem Falle kann man die durch Reaktion des Trägerkristalls mit Alkali- oder Erdalkalicarbonaten gebildete, alkalische Oberfläche nochmals ansäuern, indem man in einem weiteren Schritt Säurelösungen oder wenigstens eine pulverisierte Säure, insbesondere die schwer lösliche Fumarsäure aufbringt. Auch in diesem Falle kann eine Zwischenschicht aus Gluconsäure-delta-lacton eine kohärente Schichtbildung der Säure verbessern.

Da derart hergestellte Trägersysteme in sich ausserordentlich stabil sind, sind sie auch sehr feuchtigkeitsunempfindlich, weil die Säureoberfläche quasi passiviert ist; sie reagieren nicht mit Luftfeuchtigkeit, sondern erst dann, wenn Wasser dazutritt.

Während Systeme des Standes der Technik bei 90% Luftfeuchtigkeit bereits nach 5 bis 10 Minuten deutlich unter Brausebildung zu reagieren beginnen, sind Systeme des erfindungsgemässen Aufbaus bis zu 10 Stunden bei 90% Luftfeuchtigkeit stabil, ohne Blasen oder Reaktionsbildung zu zeigen.

Das Gluconsäure-delta-lacton ist besonders gut (1 Teil Gluconsäure-delta-lacton in 0,5 Teilen Wasser!) löslich, speziell bei höheren Temperaturen. Dieses Lacton hydrolisiert durch Wasser zu Gluconsäure, wahrscheinlich besonders dann, wenn es in wässeriger Lösung mit Alkalien zusammentrifft. Es wirkt auch als neutrales Granulierungsmittel für fertigbeschichtete Trägerkristalle, z.B. in Mischung mit Natriumcarbonat; im Kontakt mit Wasser, insbesondere bei Anwesenheit von Alkalien, wandelt es sich in reaktive Gluconsäure um.

Treibt man nun die Reaktion der Bestandteile des Brausesystems in der vorher angegebenen Weise weiter (insbesondere, wenn das sich dabei bildende Salz, z.B. Mononatriumcitrat, in kristallwasserfreier Form entsteht, wie z.B. dann, wenn die Reaktion im Vakuum abläuft), dann ergibt sich durch die Passivierung eine hohe Lagerstabilität, und zwar nicht nur wegen der Maskierung der Zitronensäure, sondern auch infolge der "inneren" Feuchtigkeitsbindekapazität des entstehenden kristallwasserfreien Salzes.

Nach dem erfindungsgemässen Verfahren lässt man Alkalicarbonate oder -bicarbonate an der Oberfläche der Trägerkörner ziemlich weit durchreagieren, vorzugsweise in der Grössenordnung von 10 bis 40%. Bei der Herstellung der Brausebasis kann man mit einem Reaktionsverlust von 5 bis 10%, maximal jedoch 15% rechnen, wobei das entstehende $CO_2$ und Wasser mittels Vakuum entfernt werden. Dieser Reaktionsverlust von 5, bzw. 10% der gesamt eingesetzten Zitronensäure, Bicarbonat- und Carbonatmengen bedeutet bei 5%, dass 16.8%, bei 10% Reaktionsverlust 33.5% der gesamten eingesetzten Menge an Brausebestandteilen als Monocitrat vorliegen. Dies würde nämlich im allgemeinen zu langsam sich auflösenden Brausetabletten führen, da das dadurch gebildete Natriumsalz als Puffersystem die Auflösung verlangsamt, nicht aber dann, wenn erfindungsgemäss Fremdsäuren eingebaut wurden.

Bringt man beispielsweise auf 900 Gewichtsteile eines Zitronensäureträgers, vorzugsweise auf Zitronensäurekristalle in der Korngrösse 0,1 bis 0,6 mm, eine konzentrierte Lösung von 100 Gewichtsteilen Äpfelsäure auf und lässt bei erhöhten Temperaturen, beispielsweise bei 60°C, diese Äpfelsäurelösung in die Oberfläche der Zitronensäure eindringen, bringt dann auf diese Oberfläche 10 bis 20 Gewichtsteile Natriumbicarbonat pulvis auf, und lässt diese bis zum Aufhören der Gasentwicklung reagieren, dann kann man die restliche Menge Natriumbicarbonat, z.B. 400 Gewichtsteile, weiterhin mit dem noch feuchten und dementsprechend klebrigen Material anreagieren lassen und trocknen.

Presst man dieses System zu Tabletten, ergeben sich Härten von 12 bis 20 kp, bei einem Tablettendurchmesser von 18 bis 20 mm, wobei die Auflösungsgeschwindigkeit etwa 30 bis 40 Sekunden beträgt.

Die erzielbaren aussergewöhnlichen Härten sind physikalisch erklärlich: Die Tragersäure wird durch die aufgebrachte konzentrierte Zweitsäure an der Oberfläche in ihrem Kristallgitter gestört, wobei sich eine oberflächliche Schmelzpunktdepression ergibt. Diese Kristallstörung wird noch vergrössert, wenn man in dieses oberflächliche Gemisch durch Reaktion Alkali-Ionen einbringt. Es hat sich gezeigt, dass derart an der Oberfläche behandelte Trägersäure-Kristalle beim Pressen der Tablette ausserordentlich hohe Bindekräfte haben, die es dann auch erlauben, schwer pressbare Wirkstoffe mitzuverpressen und auch ohne weitere Bindemittel noch genügende Tablettenhärten zu erzielen. Man kann also bei einem solchen System auf jedes Bindemittel verzichten. Trotzdem ermöglicht andererseits dieses System den Einsatz von pharmazeutischen Füll- oder Hilfsstoffen, wie z.B. Zucker, Sorbit, Mannitol oder dergleichen, soferne sie aus bestimmten Gründen erwünscht sein sollten, z.B. auch um die Feuchtigkeitsempfindlichkeit der Brausetabletten zu reduzieren, ohne dass dadurch die Auflösungszeiten der Tabletten wesentlich verlängert würden. Schliesslich verhindert der inerte Füllstoff die meist unerwünschte Interaktion des Wirkstoffs mit reaktiven Bestandteilen des Brausesystems.

Die schnelle Auflösung solcher Systeme ergibt sich aus zwei Momenten:

1.) Die Alkalibicarbonat- oder -carbonatteilchen werden eng mit der reagierten Oberfläche der Säurekristalle verbunden, so dass sich nach dem Trocknen eine Übergangsschicht von reinen Säurekristallen über die Reaktionsschichte zum reinen Alkalicarbonat oder -bicarbonat ergibt. Gelangt ein derartiges System ins Wasser, dann würde es beim Vorhandensein gleich grosser Körner einer Säure allein in der Bindeschicht zur Ausbildung eines Puffersystems kommen, das die weitere Reaktion zwischen innerer und äusserer Schichte verzögert. Man kann bei vielen handelsüblichen Brausetabletten beobachten, dass nach dem anfänglichen Auflösen der Tablette ein Kern bleibt, der nur mehr sehr langsam weiter reagiert. Das rührt daher, dass die entstehende Salz-, beispielsweise Natriumcitrat-Lösung, als Puffer zwischen den Teilchen der Brausetablette deren weitere Reaktion behindert.

2.) Die entstehende Pufferlösung wird stets dadurch gestört, dass verschieden grosse Trägerkörner sich verschieden rasch auflösen und/oder verschiedene Säuren mit verschiedenen pH-oder pK-Werten die Ausbildung eines statischen Puffers verhindern. Der pH-Wert in den Grenzschichten wird dauernd verändert und gestört, da sich zwei oder mehrere Säuren mit verschiedenen pK-Werten gegenseitig beeinflussen.

Das System lässt sich noch weiter verbessern, wenn man die Zitronensäure nach dem Anfeuchten mittels beispielsweise Äpfelsäurelösung zusätzlich mit einer pulverisierten dritten Säure abdeckt, vorzugsweise mit wenigstens einer weniger feuchtigkeitsempfindlichen Verbindung, wie z.B. Weinsäure, Fumarsäure oder Adipinsäure. Auch diese Struktur lässt man dann mit Alkalicarbonaten oder -bicarbonaten partiell durchreagieren, wobei sich im Inneren des Systems Natriumcitrate, Natriummalate etc. bilden, während an der äusseren Hülle des Partikels Natriumfumarat, Natriumadipat oder Natriumtartrat entsteht, welche meist ganz besonders unempfindlich gegen Luftfeuchtigkeit sind. Auch diese Systeme zeigen nach kurzer Zeit im Wasser raschen Zerfall und sind daher ausserordentlich gut geeignet, mit verschiedenen Wirkstoffen gemischt zu werden.

Grundsätzlich kann zwar jede feste, essbare, organische Säure als Trägerkristall verwendet werden. Jedoch ist Zitronensäure bevorzugt; überwiegend Weinsäure einzusetzen, käme zu teuer; Äpfelsäure alleine als Trägerkristall erfordert vor der Weiterverarbeitung das Abkühlen des Granulates, was in der Produktion einen zusätzlichen Schritt bedeutet.

Diese, aus zwei oder drei essbaren Säuren bestehende Struktur wird sodann mit Alkalicarbonaten zur Teilreaktion gebracht und anschliessend mit weiteren Alkalicarbonat- und/oder -bicarbonatmengen abgedeckt. Man geht dabei so vor, dass man nur einen Teil des z.B. Natriumbicarbonates hinzufügt und - bis sich kein Gas mehr entwickelt - zum Mononatriumcitrat umsetzt. Anschliessend erst wird das restliche Natriumbicarbonat und/oder Kaliumbicarbonat hinzugefügt und durch Anreaktion (teilweise Umsetzung in das Monocitrat bzw. -malat bzw. -tartrat) an die Oberfläche gebunden. Es entstehen dadurch die verschiedensten Alkalisalze von zwei bis drei verschiedenen organischen Säuren in der Grössenordnung von 5 bis 15% des Gesamtbrausesystems.

Es ist zwar nicht zwingend, dass man das Bicarbonat in zwei Teilen aufbringt, jedoch erleichtert es das Verfahren, da man durch den Einsatz einer bestimmten Menge an Natriumbicarbonat, das man völlig zu Mononatriumcitrat umsetzen lässt, einfacher die Reaktion steuern und somit auch definierter ablaufen lassen kann als dies bei dem Gesamteinsatz der Alkalien mittels alleiniger Anreaktion möglich ist. Die Menge des mit Säure umgesetzten Alkalibicarbonates oder - carbonates bewegt sich, berechnet auf die Gesamtmenge der eingesetzten Alkalien, zwischen 2 und 20%. Durchschnittlich werden 5 bis 10% der eingesetzten Alkalien im ersten Schritt umgesetzt. Hingegen werden beim späteren Beispiel 8 (Paracetamol) dadurch, dass hier sehr viel Natriumbicarbonat zum Einsatz kommt, nur 2% der eingesetzten Alkalien im ersten Schritt umgesetzt, andererseits beim späteren Beispiel 5 (Minoxidil) nahezu 20%. Dies ist einerseits wirkstoff-, andererseits pH-bedingt. Es ist aber auch ohne weiteres möglich, im Paracetamol-Beispiel die Menge des Bicarbonates für den ersten Schritt zu verdoppeln.

Immerhin sind vorzugsweise mehr Alkalien vorhanden als zur Bildung des Monosalzes nötig sind, und zwar in denjenigen Fällen, wo der pH-Wert über demjenigen des Mononatriumcitrats liegen soll, der sich bei 3,9 bis 4,15 bewegt; Brausetabletten sollen aufgelöst einen pH-Wert von 4,3 bis 4,5 oder sogar mehr aufweisen.

Abschliessend kann man bei alkaliempfindlichen Wirksubstanzen noch mittels einer Gloconsäure-delta-lacton-Lösung das Granulat befeuchten und dann entweder mit Zitronensäure oder Fumarsäure abdecken, um aussen eine saure Schicht zu erhalten. In anderen Fällen wird man abschliessend mit Gluconsäure-delta-lactonund wasserfreiem Natriumcarbonat granulieren. Bei Substanzen, die nicht alkaliempfindlich sind, kann der letzte Schritt, das Versetzen mit Gluconsäure-delta-lacton-Lösung und das abschliessende Abdecken mit Zitronensäure entfallen.

Bei den folgenden Beispielen handelt es sich um drei grundsätzlich voneinander differente Systeme. Entweder ist der Wirkstoff alkaliempfindlich und das Natriumbicarbonat muss durch Anreaktion sehr fest an die Zitronensäure gebunden werden; dazu gehören Acetylcystein, Captopril (bei dem die Basis noch zusätzlich mit Zitronensäure abgedeckt wird) und auch das Minoxidil. Oder die Wirkstoffe sind speziell feuchtigkeitsempfindlich; dazu gehören Ambroxol, Ranitidin und Cimetidin. Wirkstoffe, von denen sehr hohe Mengen in einer kleinen Tablette untergebracht werden müssen, sind Sucralfat, Ascorbinsäure, Argininaspartat und Mesna.

Brausesysteme für das Argininaspartat und das Sucralfat, bei denen es sich um neutrale Substanzen handelt, sind natürlich etwas anders aufgebaut als für das Paracetamol, das ja alkalisch ist. Im Gegensatz dazu steht das Aspirin, das einen sauren pH-Wert aufweist.

Das erfindungsgemässe Verfahren ermöglicht geradezu, insbesondere unter Einsatz von Vakuum-Misch- bzw. Granuliertrommeln, Brausesysteme auf den jeweiligen Wirkstoff und seine Menge genauestens abzustimmen bzw. sogar vorzuprogrammieren und gegebenenfalls die einzelnen Schritte sogar computergesteuert ablaufen zu lassen.

Beispiel 1 (Acetylcystein):

200 mg des Wirkstoffes Acetylcystein werden in einer 1,3 g Tablette untergebracht. Das Acetylcystein ist empfindlich gegen Alkalien und wird durch alkalische Oxydation (Interaktion mit z.B. Natriumbicarbonat; gegenüber Natriumcarbonat ist es relativ stabil!) in das instabile Dimer übergeführt. Um dies zu verhindern, wird das Natriumbicarbonat durch Reaktion und teilweise Umsetzung in das Natriumcitrat sehr stark an die Zitronensäure angebunden, um möglichst wenig freies Natriumbicarbonat vorliegen zu haben, das mit dem sauren Acetylcystein in Interaktion treten kann.

| Teile: | |
|---|---|
| 379 | Kristallisierte Zitronensäure |
| 100 | Äpfelsäure |
| 105 | Zitronensäure pulvis |
| 20 | Natriumbicarbonat I |
| 180 | Natriumbicarbonat II |
| 100 | Natriumcarbonat |
| 6 | Gluconsäure-delta-lacton für Lösung |
| 890 | |
| 200 | Acetylcystein |
| 156 | Zusatzstoffe |
| 1246 | |

Verfahren: Zitronensäure und Äpfelsäure aufheizen auf 60°C und mit 6 ml einer Gluconsäure-delta-lacton-Lösung (10 Teile Gluconsäure-delta-lacton, 5 Teile Wasser) befeuchten. Natriumbicarbonat I zugeben und zu Mononatriumcitrat umsetzen lassen; darauf Natriumbicarbonat II anreagieren lassen (teilweise Umsetzung zum Citrat). Zitronensäure pulvis zugeben und die Oberfläche durch Anreaktion abdecken. Natriumcarbonat zugeben und anreagieren lassen, abschliessend vakuumtrocknen. Diese Grund-Brausebasis wird mit 200 Teilen Acetylcystein und mit entsprechenden Füll-, Aroma- und Süss-Stoffen vermischt.

Beispiel 2 (Acetylcystein):

| Teile | |
|---|---|
| 275 | Kristallisierte Zitronensäure |
| 45 | Weinsäure |
| 75 | Zitronensäure pulvis |
| 20 | Natriumbicarbonat I |
| 240 | Natriumbicarbonat II |
| 138 | Natriumcarbonat |
| 12 | Mononatriumcitrat in 30%iger wässeriger Lösung |
| 805 | |
| 600 | Acetylcystein |
| 215 | Zusatzstoffe |
| 1620 | |

Verfahren: Kristallisierte Zitronensäure und Weinsäure aufheizen auf 50°C; Mononatriumcitratlösung eindringen lassen, mit Zitronensäure pulvis abdecken; anschliessend Natriumbicarbonat I zugeben und zu Mononatriumcitrat umsetzen; sodann den Rest Natriumbicarbonat zufügen und anreagieren lassen (teilweise Umsetzung zum Citrat); abschliessend Natriumcarbonat zufügen und anreagieren lassen. Zu diesem Grundgranulat werden 600 Teile Acetylcystein sowie Zusatzstoffe, Aroma- und Süss-Stoffe zugemischt.

Beispiel 3 (Ambroxol):

Das temperatur- und feuchtigkeitsempfindliche Ambroxol wird mittels Äpfelsäure-Lösung an eine stark passivierte und dadurch unempfindliche Basis von Trägerkristallen aus Zitronensäure und Weinsäure gebunden.

| Teile | |
|---|---|
| 515 | Kristallisierte Zitronensäure |
| 170 | Weinsäure |
| 25 | Natriumbicarbonat I |
| 325 | Natriumbicarbonat II |
| 60 | Natriumcarbonat |
| 6 | Äpfelsäure für Lösung I |
| 1101 | |
| 30 | Ambroxol-HCl |
| 244 | Zusatzstoffe ad |
| 1375 | |

Verfahren: Kristallisierte Zitronensäure und Weinsäure aufheizen auf 60°C, mit 8 ml 60%iger Äpfelsäurelösung befeuchten. Natriumbicarbonat I bis zur Bildung von Mononatriumcitrat durchreagieren lassen, anschliessend Natriumbicarbonat II anreagieren lassen (teilweise Umsetzung zu Citrat). Natriumcarbonat zugeben, anreagieren lassen und vakuumtrocknen. Das erzielte Granulat mit Ambroxol-HCl und den Zusatzstoffen (Süss-Stoff, Aroma) vermischen und zu Tabletten verpressen.

Beispiel 4 (Captopril):

Captopril ist aufgrund seiner chemischen Struktur in noch stärkerem Masse als das Acetylcystein empfindlich gegen alkalische Substanzen, unter deren Einfluss es das instabile Dimer bildet. Hier reicht das Binden der Bicarbonate an die Zitronensäure allein nicht aus, und es muss die Brausebasis noch durch eine Schicht pulverisierter Zitronensäure oder mittels Zitronensäure-Lösung bzw. Gluconsäure-delta-lacton-Lösung entschärft werden.

| Teile | |
|-------|---|
| 250 | Kristallisierte Zitronensäure |
| 55 | Mononatriumcitrat |
| 67 | Äpfelsäure |
| 63 | Fumarsäure |
| 11 | Natriumbicarbonat I |
| 173 | Calciumcarbonat |
| 36 | Natriumcarbonat |
| 10 | Gluconsäure-delta-lacton |
| 665 | |
| 25 | Captopril |
| 110 | Zusatzstoffe |
| 800 | |

Verfahren: Kristallisierte Zitronensäure, Mononatriumcitrat und Äpfelsäure aufheizen auf 60 °C. Befeuchten mit 10 ml Gluconsäure-delta-lacton-Lösung (10 Teile Gluconsäure-delta-lacton, 5 Teile Wasser). Natriumbicarbonat I zugeben und durchreagieren lassen bis zur Bildung von Mononatriumcitrat; dann Natriumcarbonat und Calciumcarbonat zugeben und reagieren lassen; anschliessend Fumarsäure pulvis dazugeben und die Oberfläche durch Anreaktion abdecken; anschliessend vakuumtrocknen. Diese Grundbasis wird mit 25 Teilen Captopril, den Zusatzstoffen, wie z.B. Süss-Stoffen, Aroma- und allfälligen Füllstoffen vermischt.

Beispiel 5 (Minoxidil):

| Teile | |
|-------|---|
| 243 | Zitronensäure krist. |
| 80 | Äpfelsäure |
| 120 | Zitronensäure pulvis |
| 39 | Natriumbicarbonat I |
| 61 | Natriumbicarbonat II |
| 79 | Kaliumhydrogencarbonat |
| 70 | Natriumcarbonat |
| 10 | Gluconsäure-delta-lacton für Lösung |
| 702 | |
| 10 | Minoxidil |
| 38 | Zusatzstoffe |
| 750 | |

Verfahren: Kristallisierte Zitronensäure und Äpfelsäure werden auf 60 °C aufgeheizt, mit 10 ml Gluconsäure-delta-lacton-Lösung (10 Teile Gluconsäure-delta-lacton auf 5 Teile Wasser) befeuchtet; Natriumbicarbonat I zugeben und bis zur Bildung von Mononatriumcitrat durchreagieren lassen. Anschliessend Natriumbicarbonat II und Kaliumbicarbonat zufügen und anreagieren lassen (partielle Umsetzung zum Citrat). Anschliessend wird Zitronensäure pulvis zugegeben und die Oberfläche durch Anreaktion abgedeckt. Abschliessend wird Natriumcarbonat zugegeben und anreagieren gelassen, schliesslich im Vakuum getrocknet. Diese Grund-Brausebasis wird mit 10 Teilen Minoxidil und mit den Zusatzstoffen (z.B. Süss-Stoffen, sowie Aroma) vermischt.

Beispiel 6 (Ranitidin):

| Teile | |
|---|---|
| 630 | Zitronensäure krist. |
| 210 | Weinsäure |
| 210 | Zitronensäure pulvis |
| 370 | Mononatriumcitrat |
| 40 | Natriumbicarbonat I |
| 460 | Natriumbicarbonat II |
| 100 | Natriumcarbonat |
| 16 | Gluconsäure-delta-lacton für Lösung |
| 2036 | |
| 170 | Ranitidin-HCl |
| 294 | Zusatzstoffe |
| 2500 | |

Verfahren: Kristallisierte Zitronensäure, Weinsäure und Mononatriumcitrat aufheizen auf 60°C. Befeuchten mit 16 ml einer Gluconsäure-delta-lacton-Lösung (10 Teile Gluconsäure-delta-lacton, 5 Teile Wasser). Natriumbicarbonat I zugeben, das komplett zu Mononatriumcitrat umgesetzt wird.

Anschliessend Natriumbicarbonat II anreagieren lassen (teilweise Umsetzung zum Citrat). Zitronensäure pulvis dazugeben und die Oberfläche durch Anreaktion abdecken. Abschliessend Natriumcarbonat anreagieren lassen und im Vakuum trocknen. Diese Grund-Brausebasis wird mit 170 Teilen Ranitidin-HCl und entsprechenden Zusatzstoffen, wie Süss-Stoff und Aroma, vermischt.

Beispiel 7 (Cimetidin):

200 mg Wirkstoff werden in einer 2,3g-Tablette untergebracht:

| Teile | |
|---|---|
| 706 | Kristallisierte Zitronensäure |
| 200 | Äpfelsäure |
| 250 | Zitronensäure pulvis |
| 16 | Natriumbicarbonat I |
| 462 | Natriumbicarbonat II |
| 102 | Natriumcarbonat |
| 6 | Zitronensäure in 50%iger wässeriger Lösung |
| 1742 | |
| 200 | Cimetidin |
| 368 | Zusatzstoffe |
| 2310 | |

Verfahren: Kristallisierte Zitronensäure und Äpfelsäure auf 60°C aufheizen, mit 6 ml 50%iger Zitronensäure-Lösung befeuchten, Nariumbicarbonat I zugeben und zum Mononatriumcitrat durchreagieren lassen. Dann Natriumbicarbonat II anreagieren lassen (teilweise Umsetzung zum Citrat). Die Oberfläche durch Anreaktion mit Zitronensäure pulvis abdecken; abschliessend Natriumcarbonat anreagieren lassen und im Vakuum trocknen. Diese Grund-Brausebasis wird mit 200 Teilen Cimetidin und entsprechenden Zusatzstoffen (Füll, Aroma- und Süss-Stoffen) vermischt.

Beispiel 8 (Paracetamol):

500 mg Paracetamol können erfindungsgemäss in einer 2,74 g Tablette mit guten Auflösungseigenschaften eingebaut werden.

Das Problem bei der Paracetamol-Brausetablette ist, dass das Paracetamol alkalisch reagiert und der bevorzugte pH-Wert der trinkfertigen Lösung ca. 6 beträgt, so dass ein Überschuss an alkalischen Substanzen, wie Bicarbonat, bzw. Carbonat, eine schnelle Auflösung verhindert, da relativ wenig Zitronensäure vorhanden ist und somit die Bildung eines Wirkstoffkernes, der sich schlecht löst, begünstigt wird. Durch das neue Granulierungsprinzip ist es möglich, diese Nachteile zu beseitigen.

| Variante I | | | Variante II | | |
|---|---|---|---|---|---|
| Teile | | | Teile | | |
| 765 | Kristallisierte Zitronensäure | | 415 | Äpfelsäure | |
| 150 | Äpfelsäure | | 500 | Kri stall.Zitronensäure | |
| 20 | Natriumbicarbonat I | | 20 | Natriumbicarbonat I | |
| 913 | Natriumbicarbonat II | | 913 | Natriumbicarbonat II | |
| 150 | Natriumcarbonat | | 150 | Natriumcarbonat | |
| 25 | Gluconsäure-delta-lacton für Lösung I | | 25 | GDL für Lösung I | |
| 2023 | | | 2023 | | |
| 500 | Paracetamol | | 500 | Paracetamol | |
| 50 | Gluconsäure-delta-lacton für Lösung II | | 50 | GDL für Lösung II | |
| 167 | Zusatzstoffe | | 167 | Zusatzstoffe | |
| 2740 | | | 2740 | | |

Verfahren: Kristallisierte Zitronensäure und Äpfelsäure auf 50°C aufheizen und mit 25 ml Gluconsäure-delta-lacton-Lösung (10 Teile Gluconsäure-delta-lacton auf 5 Teile Wasser) befeuchten (die Zitronensäure wird dotiert); Natriumbicarbonat I zum Mononatriumcitrat durchreagieren lassen; darauf Natriumbicarbonat II anreagieren lassen (teilweise Umsetzung zum Citrat); danach Natriumcarbonat etwas anreagieren lassen. Anschliessend Paracetamol zugeben. Mit 50 ml GDL-Lösung (10 Teile Gluconsäure-delta-lacton auf 5 Teile Wasser) granulieren. Anschliessend im Vakuum trocknen. Das erzielte Grundgranulat mit entsprechenden Zusatzstoffen, wie Süss-Stoffen und Aroma, vermischen.

Beispiel 9 (Acetylsalicylsäure):

Eine 2,6g-Tablette enthält 500 mg Acetylsalicylsäure und, um einen reduzierten Natriumgehalt zu erreichen, als Alkalien zusätzlich Kalium- und Calciumsalze. Da das saure Aspirin zur Interaktion mit den Alkalicarbonaten neigt, wurde die Grundbasis mit Zitronensäure abgedeckt.

| Teile | |
|---|---|
| 259 | Kristallisierte Zitronensäure |
| 130 | Äpfelsäure krist. |
| 264 | Zitronensäure pulvis |
| 15 | Natriumbicarbonat I |
| 466 | Natriumbicarbonat II |
| 46 | Calciumcarbonat |
| 137 | Kaliumhydrogencarbonat |
| 132 | Kaliumcarbonat |
| 42 | Natriumcarbonat |
| 27 | Gluconsäure-delta-lacton für Lösung |
| 1518 | |
| 500 | Aspirin |
| 47 | Zusatzstoffe |
| 2065 | |

Verfahren: Kristallisierte Zitronensäure aufbringen, anschliessend auf 60°C aufheizen und mit 36 ml Gluconsäure-delta-lacton-Lösung (10 Teile Gluconsäure-delta-lacton auf 5 Teile Wasser) benetzen; Äpfelsäure aufbringen, anschliessend Natriumbicarbonat I zum Mononatriumcitrat bzw. -malat durchreagieren lassen, darauf Natriumbicarbonat II, Calciumcarbonat und Kaliumbicarbonat anreagieren lassen (teilweise Umsetzung zum Citrat). Anschliessend die Oberfläche mit Zitronensäure pulvis abdecken. Abschliessend Natriumcarbonat anreagieren lassen und im Vakuum trocknen. Diese Grund-Brausebasis wird mit 500 Teilen Aspirin und mit entsprechenden Zusatzstoffen vermischt.

Beispiel 10 (Argininaspartat):

Argininaspartat ist besonders leicht löslich und verhindert in gängigen Brausesystemen durch Ausbildung von konzentrierten Lösungen das schnelle Brausen der Tablette.

| Teile | |
|---|---|
| 384 | Kristallisierte Zitronensäure |
| 53 | Natriumbicarbonat I |
| 144 | Natriumbicarbonat II |
| 88 | Kaliumbicarbonat |
| 61 | Natriumcarbonat |
| 6 | Äpfelsäure für Lösung I |
| 736 | |
| 1000 | Argininaspartat |
| 41 | Zusatzstoffe |
| 1777 | |

Verfahren: Zitronensäure aufheizen auf 60°C, mit 8 ml 60%iger Äpfelsäurelösung befeuchten. Natriumbicarbonat I zu Mononatriumcitrat bzw. -malat durchreagieren lassen; anschliessend Natriumbicarbonat II und Kaliumbicarbonat anreagieren lassen. Natriumcarbonat zugeben und anreagieren lassen, anschliessend im Vakuum trocknen. Das erzielte Granulat mit Argininaspartat und den Zusatzstoffen (Süss-Stoff, Aroma) vermischen und zu Tabletten verpressen.

Beispiel 11 (Sucralfat):

Bei der Herstellung einer Sucralfat-Tablette steht sehr wenig Natriumbicarbonat zur Verfügung. Es muss daher gemeinsam mit dem zweiten Bicarbonat mittels Gluconsäure-delta-lacton-Lösung granuliert werden.

| Teile | |
|---|---|
| 394 | Kristallisierte Zitronensäure |
| 42 | Natriumbicarbonat I |
| 158 | Natriumbicarbonat II |
| 6 | Äpfelsäure für Lösung |
| 200 | Gluconsäure-delta-lacton für Lösung |
| 800 | |
| 1000 | Sucralfat |
| 100 | Zusatzstoffe |
| 1900 | |

Verfahren: Kristallisierte Zitronensäure auf 60°C aufheizen und mit 8 ml 60%iger ÄpfelsäureLösung benetzen. Natriumbicarbonat I zu Mononatriumcitrat durchreagieren lassen, Sucralfat und Natriumbicarbonat II zugeben und mit 280 ml Gluconsäure-delta-lacton-Lösung (10 Teile Gluconsäure-delta-lacton/3 Teile Wasser/3 Teile Äthanol) granulieren, anschliessend im Vakuuum trocknen. Das Granulat mit den Zusatzstoffen (Süss-Stoff, Aroma) vermischen.

Beispiel 12 (Mesna):

| Teile | |
|---|---|
| 610 | Kristallisierte Zitronensäure |
| 40 | Weinsäure |
| 34 | Natriumbicarbonat I |
| 236 | Natriumbicarbonat II |
| 68 | Natriumcarbonat |
| 12 | Äpfelsäure für Lösung I |
| 1000 | |
| 1000 | Mesna |
| 78 | Zusatzstoffe |
| 2078 | |

Verfahren: Kristallisierte Zitronensäure auf 60°C aufheizen, mit 14 ml 70%iger Äpfelsäurelösung benetzen, Weinsäure aufbringen und eindringen lassen. Natriumbicarbonat I zum Mononatriumcitrat bzw.-malat durchreagieren lassen; Natriumbicarbonat II zugeben und anreagieren lassen (teilweise Umsetzung zum Citrat). Natriumcarbonat zugeben und vakuumtrocknen. Mesna wird mit einer 30%igen Sorbitol-Lösung in Alkohol/Wasser 1:1 granuliert, getrocknet, gesiebt und mit der gemäss Beispiel hergestellten Brausebasis, sowie allenfalls gewünschten Zusatzstoffen vermischt und zu Tabletten verpresst.

Beispiel 13 (Vitamin C):

| Teile | |
|---|---|
| 1020 | Ascorbinsäure |
| 588 | Kristallisierte Zitronensäure |
| 50 | Natriumbicarbonat I |
| 350 | Natriumbicarbonat II |
| 140 | Natriumcarbonat |
| 12 | Äpfelsäure für Lösung |
| 2160 | |
| 40 | Zusatzstoffe |
| 2200 | |

Verfahren: Kristallisierte Zitronensäure und Ascorbinsäure aufheizen auf 60°C. Mit 16 ml 60%iger Äpfelsäure-Lösung befeuchten. Natriumbicarbonat I zu Mononatriumcitrat bzw.-malat durchreagieren lassen; danach Natriumbicarbonat II anreagieren lassen. Natriumcarbonat zugeben und anreagieren lassen. Anschliessend im Vakuuum trocknen. Das erzielte Granulat mit den Zusatzstoffen (Süss-Stoff, Aroma) vermischen.

In der folgenden Tabelle werden die Beispiele 1 bis 13 übersichtlich zusammengefasst. Es bedeuten:

CA      Zitronensäure (in Trägerkristallen als Feingries, in den Schichten als Pulver)
MA      Äpfelsäure
FA      Fumarsäure
TA      Weinsäure
CC      Calciumcarbonat
SC      Natriumcarbonat
KH      Kaliumbicarbonat
MNC      Mononatriumcitrat
GDL      Gluconsäure-delta-lacton (67 %ige Lösung)
BC1      Natriumbicarbonat 1.Teil(wird zum Monosalz durchreagiert)
BC2      Natriumbicarbonat 2. Teil (wird nur durch Anreaktion auf der vorhergehenden Schichte verankert)

| Schicht auf den Trägerkristallen | | 1 | 2 | 3 | 4 | 5 | 6 | 7 |
|---|---|---|---|---|---|---|---|---|
| Beispl. | Trägerkristalle | | | | | | Wirkstoff | |
| 1 | CA,MA | GDL | BC1 | BC2 | CA | SC | Acetylcystein | |
| 2 | CA,TA | MNC | CA | BC1 | BC2 | SC | Acetylcystein | |
| 3 | CA,TA | MA | BC1 | BC2 | SC | | Ambroxol | |
| 4 | CA,MNC,MA | GDL | BC | CC | FA | SC | Captopril | |
| 5 | CA,MA | GDL | BC1 | BC2 KH | CA | SC | Minoxidil | |
| 6 | CA,TA,MNC | GDL | BC1 | BC2 | CA | SC | Ranitidin | |
| 7 | CA,MA | CA | BC1 | BC2 | CA | SC | Cimetidin | |
| 8 | CA,MA | GDL | BC1 | BC2 | SC | | Paracetamol | GDL |
| 9 | CA | GDL | MA | BC1 | BC2 CC,KH | CA SC | Acetylsalicylsäure | |
| 10 | CA | MA | BC1 | BC2 | KH | SC | Argininaspartat | |
| 11 | CA,MA | BC1 | BC2 | GDL | | | Sucralfat | |
| 12 | CA,MA | TA | BC1 | BC2 | SC | | Mesna | |
| 13 | CA Vit.C,MA | BC1 | BC2 | SC | | | | |

Der Wirkstoff wird - ausser in den Beispielen 8, 11 und 13 - mit der fertigen Brausebasis gemischt und zu Tabletten verpresst. In den Beispielen 8 und 11 wird der Wirkstoff mit einem oder mehreren anderen Schichtbestandteil(en) gemischt und als Schicht aufgetragen; im Beispiel 13 wird der Wirkstoff (Vitamin C = Ascorbinsäure) mit Zitronensäure als Trägerkristall vorgelegt.

Beispiel 14 (Argininaspartat und Acetylcystein):

Es folgt eine Tabelle über die Variation der verschiedenen Brausebasen. Folgende fünf Brausebasen wurden nach dem erfindungsgemässen Prinzip hergestellt:

| Brausebasis | a | b | c | d | e |
|---|---|---|---|---|---|
| Zitronensäure Feingries | 900 | | | | 900 |
| Zitronensäure für Lösung | | 19 | 19 | 19 | 24 |
| Weinsäure Typ 2V | | 900 | 100 | | |
| Weinsäure pulvis | 100 | | | | |
| NaHCO$_3$ (BC1) | 50 | 50 | 50 | 50 | |
| NaHCO$_3$ (BC2) | 350 | 350 | 350 | 350 | |
| Na$_2$CO$_3$ | 100 | 100 | 120 | 270 | 100 |
| Äpfelsäure Feingries | | 100 | 900 | 900 | |
| Äpfelsäure für Lösung | 19 | | | | |
| Adipinsäure | | | | 100 | |
| Fumarsäure | | | | | 100 |
| KHCO$_3$ | | | | | 150 |
| K$_2$CO$_3$ | | | | | 250 |

Diese fünf Brausebasen a bis e wurden in alternierenden Mengen mit jeweils 1000 mg Argininaspartat, bzw. mit 1000 mg Acetylcystein und 290 mg Na$_2$CO$_3$ gemischt und zu Tabletten verpresst. Die dabei resultierenden Tablettenhärten Auflösungszeiten in nur 50 ml Wasser (!) und der dabei erzielte pH-Wert werden angegeben:

| Brausebasis | 1000 mg Argininaspartat | | | 1000mg Acetylcystein | |
| | 500mg | 750mg | 1000mg | 460 mg | 710 mg |
| --- | --- | --- | --- | --- | --- |
| a Härte | 5,9 | 6,3 | 6,5 | 6-7 | 6-7 |
| Auflöszeit | 50" | 50" | 60" | 70" | 75" |
| pH-Wert | 4,5 | 4,45 | 4,35 | 4,3 | 4,4 |
| b Härte | 6,1 | 5,9 | 7,6 | 7,1 | 7,5 |
| Auflöszeit | 50" | 70" | 100" | 100" | 115" |
| pH-Wert | 4,3 | 4,1 | 4,1 | 4,01 | 4,1 |
| c Härte | 6,0 | 6,2 | 7,2 | 6-7 | 6-7 |
| Auflöszeit | 50" | 50" | 75" | 90" | 80" |
| pH-Wert | 4,5 | 4,4 | 4,3 | 4,1 | 4,3 |
| d Härte | 5,9 | 7,2 | 6,2 | 6-7 | 6-7 |
| Auflöszeit | 60" | 80" | 70" | 75" | 85" |
| pH-Wert | 4,99 | 4,86 | 4,9 | 5,1 | 5,1 |
| e Härte | 5,9 | 6,9 | 6,2 | 6-7 | 6-7 |
| Auflöszeit | 80" | 75" | 60" | 50" | 60" |
| pH-Wert | 4,55 | 4,5 | 4,39 | 4,4 | 4,35 |

Beispiel 15:

Dimeticon (Dimethylpolysiloxan) wird normalerweise als Anti-Schaummittel bei Suspensionen, z.B. beim Dragieren, oder teilweise auch bei Cremen und Brausetabletten-Formeln verwendet. Bisher wurde das Dimeticon auch verwendet, um die Feuchtigkeitsempfindlichkeit von Brausetabletten herabzusetzen. Erfindungsgemäss gelingt es nun aber, durch Zusatz von Dimeticon, das auf einen Träger in geringen Mengen aufgezogen wird, die Reaktionsgeschwindigkeit der Brausetablette um 20 bis 30 % zu erhöhen, d.h., die Auflöszeit zu beschleunigen. Durch das Dimeticon wird offensichtlich eine Verbesserung des Reaktionskontaktes zwischen den Brause-Reaktionspartnern erreicht, und zwar indem man geringe Mengen von Dimeticon auf einen neutralen Träger aufzieht und diesen zur Brausebasis zumischt.

Dabei geht man folgendermasse vor: 100 Gewichtsteile neutraler Füllstoffe, wie z.B. Mannitol, Sorbitol, Lactose, Maltodextrin oder Aerosil werden auf 40°C aufgeheizt. Eine Lösung von 0,1 bis 0,8 Gewichtsteilen Dimeticon in organischen Lösungsmitteln wird in eine diesen Träger enthaltende Vakuumtrommel eingesaugt. Während intensiven Rührens wird das Lösungsmittel verdampft, um eine entsprechende Gleichverteilung zu erhalten. Das so erhaltene Produkt wird der Brausetablette zugesetzt, und zwar je nach Wirkstoff - dessen Hydrophobie oder Hydrophilie hier auch eine Rolle spielt - zwischen 0,1 und 0,6 mg pro Tablette. Z.B. erreicht man bei einer Cimetidin-Brausetablette ohne Dimeticon eine Auflöszeit von 80 sec, während man bei gleicher Härte mit Dimeticon eine Auflöszeit von 55 sec erreicht.

Beim Ambroxol kann man die Auflöszeit von 120 sec durch Zugabe von 100 mg Mannit, auf das 0,4 mg Dimeticon aufgezogen ist, auf 70 bis 80 sec drücken.

**Patentansprüche**

1. Brausetablette, enthaltend wenigstens einen pharmazeutischen Wirkstoff und ein Brausesystem aus wenigstens einer festen, essbaren, organischen Säure, wenigstens einem Alkalicarbonat oder -bicarbonat als gasabspaltendem Bestandteil, und wenigstens einem Alkalisalz der Säure, wobei auf aus der wenigstens einen Säure bestehenden Trägerkristallen wenigstens zwei Schichten aufgebracht sind, dadurch gekennzeichnet, dass die erste Schicht wenigstens eine andere, feste, essbare, organische Säure und/oder das - vorzugsweise saure - Alkalisalz dieser anderen Säure, eine andere Schicht hingegen wenigstens ein - vorzugsweise saures - Alkalisalz wenigstens einer der beiden Säuren enthält.

**2.** Brausetablette nach Anspruch 1, dadurch gekennzeichnet, dass von der Gesamtmenge der eingesetzten Säure(n) und der gasabspaltenden Bestandteile 10 bis 40, insbesondere 10 bis 20 Gewichtsprozent in Form der Salze, vorzugsweise in Form saurer Salze, vorliegen.

**3.** Brausetablette nach Anspruch 1 oder 2, insbesondere für einen besonders feuchtigeitsempfindlichen Wirkstoff, wie z.B. Ambroxol, Ranitidin oder Cimetidin, dadurch gekennzeichnet,dass wenigstens ein Teil des Salzes und/oder des gasabspaltenden Bestandteils in im wesentlichen kristallwasserfreier Form vorliegt, wobei ein allfälliger, kristallwasserfreier, gasabspaltender Bestandteil vorzugsweise die äusserste Schicht der Trägerkristalle bildet.

**4.** Brausetablette nach einem der vorhergehenden Ansprüche, dadurch gekennzeichnet, dass das Brausesystem ein Säurebindungsvermögen von höchstens 5 meq aufweist.

**5.** Brausetablette nach einem der vorhergehenden Ansprüche, dadurch gekennzeichnet, dass sie 0.1 bis 0.6 mg Dimeticon enthält, das auf einem neutralen Hilfsstoff bzw. Träger, wie z.B. Mannitol, Sorbitol, Lactose, Maltodextrin oder Aerosil aufgezogen ist.

**6.** Brausetablette nach einem der vorhergehenden Ansprüche, dadurch gekennzeichnet, dass sie auf 100 Gewichtsteile des Brausesystems fünfzehn bis dreissig Gewichtsteile Füll- bzw. Hilfsstoffe, wie z.B. Mannit, Lactose oder Sorbitol enthält.

**7.** Brausetablette nach einem der vorhergehenden Ansprüche, dadurch gekennzeichnet, dass sie wenigstens zwei essbare organische Säuren mit unterschiedlichem pK-Wert, insbesondere Zitronensäure und Äpfelsäure, vorzugsweise in einem Verhältnis von 20:1 bis 3:1, insbesondere 12:1 bis 8:1, enthält.

**8.** Brausetablette nach einem der vorhergehenden Ansprüche, dadurch gekennzeichnet, dass die gasabspaltenden Bestandteile in unterstöchiometrischer Menge - vorzugsweise in einer nur das Monosalz bildenden Menge - vorliegen.

**9.** Brausetablette nach einem der vorhergehenden Ansprüche, dadurch gekennzeichnet, dass etwa 5 bis etwa 20, insbesondere 10 bis 15 Gewichtsprozent der Gesamtmenge gasabspaltender Bestandteile in einer zweiten oder dritten Schicht zum Monosalz durchreagiert vorliegen, während der Rest in der darüberliegenden Schicht nur durch Anreaktion auf dem Trägerkristall verankert ist.

**10.** Brausetablette nach einem der vorhergehenden Ansprüche, dadurch gekennzeichnet, dass in der Reihenfolge Zitronensäure - Äpfelsäure - Weinsäure - Adipinsäure eine jeweils zuerst genannte Säure im Trägerkorn vorliegt, auf dessen Oberfläche wenigstens eine nachfolgende Säure oder ihr Salz verankert - vorzugsweise in das Kristallgitter des Trägerkorns eindiffundiert - und mit wenigstens einem Teil des gasabspaltenden Bestandteils unter Bildung einer aus dem Salz bestehenden Zwischenschicht wenigstens teilweise abgedeckt ist.

**11.** Brausetablette nach einem der vorhergehenden Ansprüche, dadurch gekennzeichnet, dass als Trägerkristalle eine Mischung von Zitronensäure mit wenigstens einer der folgenden Verbindungen vorliegt: Äpfelsäure, Weinsäure, Mononatriumcitrat.

**12.** Brausetablette nach einem der vorhergehenden Ansprüche, dadurch gekennzeichnet, dass die erste Schicht - soferne die Trägerkristalle nicht ausschliesslich aus Äpfelsäure bestehen - Äpfelsäure enthält.

**13.** Brausetablette nach einem der vorhergehenden Ansprüche, mit einer ersten Schicht aus Gluconsäuredelta-lacton, dadurch gekennzeichnet, dass die erste Schicht mit wenigstens einer - vorzugsweise mit mindestens zwei der folgenden Säuren bzw. deren Alkalisalzen abgedeckt ist: Zitronensäure, Äpfelsäure, Weinsäure.

**14.** Brausetablette nach einem der vorhergehenden Ansprüche, dadurch gekennzeichnet, dass die zweite Schicht aus einer Teilmenge der Gesamtmenge des gasabspaltenden Bestandteils, wenigstens teilweise als Monosalz der die Trägerkristalle und/oder die erste Schicht bildenden Säure gebildet ist.

**15.** Brausetablette nach einem der vorhergehenden Ansprüche, mit einem alkaliempfindlichen Wirkstoff, wie z.B. Acetylcystein, Captopril oder Minoxidil, dadurch gekennzeichnet, dass die auf den Trägerkristallen aufgebrachte Schicht mit den gasabspaltenden Bestandteilen mittels einer Säure, vorzugsweise Zitronen- oder Fumarsäure abgedeckt ist.

**16.** Brausetablette nach einem der vorhergehenden Ansprüche, dadurch gekennzeichnet, dass sie - bezogen auf 100 Gewichtsteile des Brausesystems - wenigstens 50, vorzugsweise wenigstens 100 Gewichtsteile des pharmazeutischen Wirkstoffes, wie z.B. Sucralfat oder Paracetamol, enthält, wobei der Wirkstoff vorzugsweise in die die gasabspaltenden Bestandteile enthaltende Schicht eingebaut ist, und dass die so überzogenen Trägerkristalle in Mischung mit Gluconsäure-delta-lacton und/oder - insbesondere kristallwasserfreiem - Natriumcarbonat granuliert vorliegen.

**17.** Verfahren zur Herstellung eines Brausesystems aus wenigstens einer festen, essbaren, organischen Säure, wenigstens einem durch Reaktion mit der Säure unter Salzbildung gasabspaltenden Bestandteil und wenigstens einem aus der Säure und dem gasabspaltenden Bestandteil gebildeten Salz, wobei die Oberfläche der in einer Schüttung vorliegenden Kristalle der wenigstens einen Säure mit Wasser oder der Lösung eines für das Brausesystem vorgesehenen Bestandteils benetzt und wenigstens teilweise anreagiert sowie höchstens teilweise getrocknet wird, worauf jeweils ein in feinkörniger Form vorliegender, weiterer Bestandteil auf der Oberfläche verankert und - gegebenenfalls nach teilweiser Anreaktion - die Masse getrocknet wird, dadurch gekennzeichnet, dass auf der Oberfläche der benetzten Säurekristalle eine andere Säure und anschliessend wenigstens ein Teil des gasabspaltenden Bestandteils verankert werden.

**18.** Brausetablette nach einem der vorhergehenden Ansprüche, dadurch gekennzeichnet, dass sie - vorzugsweise 0,1 bis 0,6 mg - Dimeticon enthält.

**Claims**

**1.** An effervescent tablet containing at least one pharmaceutical active substance and an effervescent system comprising at least one solid, edible, organic acid, at least one alkali metal carbonate or bicarbonate as a gas-forming component and at least one alkali metal salt of the acid, at least two layers being applied to carrier crystals consisting of the one or more acids, wherein the first layer contains at least one other solid, edible, organic acid and/or the - preferably acidic - alkali metal salt of this other acid, whereas another layer contains at least one - preferably acidic - alkali metal salt of at least one of the two acids.

**2.** An effervescent tablet as claimed in claim 1, wherein, of the total amount of the acid(s) used and of the gas-forming components, 10 to 40, in particular 10 to 20, percent by weight are present in the form of the salts, preferable in the form of acidic salts.

**3.** An effervescent tablet as claimed in claim 1 or 2, in particular for an especially moisture-sensitive active substance, such as, for example, ambroxol, ranitidine or cimetidine, wherein at least a part of the salt and/or of the gas-forming component is present in essentially anhydrous form, any anhydrous, gas-forming component preferably forming the outermost layer of the carrier crystal.

**4.** An effervescent tablet as claimed in any of the preceding claims, wherein the effervescent system has an acid-consuming capacity of not more than 5 meq.

**5.** An effervescent tablet as claimed in any of the preceding claims, which contains 0.1 to 0.6 mg of dimeticon, which is applied to a neutral excipient or carrier, such as, for example, mannitol, sorbitol, lactose, maltodextrin or Aerosil.

**6.** An effervescent tablet as claimed in any of the preceding claims, which contains fifteen to thirty parts by weight of fillers or excipients, such as, for example, mannitol, lactose or sorbitol, per 100 parts by weight of the effervescent system.

**7.** An effervescent tablet as claimed in any of the preceding claims, which contains at least two edible organic acids having different pK values, in particular citric acid and malic acid, preferably in a ratio of 20:1 to 3:1, in particular 12:1 to 8:1.

8. An effervescent tablet as claimed in any of the preceding claims, wherein the gas-forming components are present in a substoichiometric amount - preferably in an amount which forms only the monosalt.

9. An effervescent tablet as claimed in any of the preceding claims, wherein about 5 to about 20, in particular 10 to 15, percent by weight of the total amount of gas-forming components have reacted to give the monosalt and are present in this form in a second or third layer, while the remainder in the layer above this is anchored to the carrier crystal only by partial reaction.

10. An effervescent tablet as claimed in any of the preceding claims, wherein, in the sequence citric acid - malic acid - tartaric acid - adipic acid, a first-mentioned acid is present in the carrier particle, on whose surface at least one subsequent acid or its salt is anchored - preferably diffused in the crystal lattice of the carrier particle - and is at least partly covered with at least a part of the gas-forming component with formation of an intermediate layer consisting of the salt.

11. An effervescent tablet as claimed in any of the preceding claims, wherein a mixture of citric acid with at least one of the following compounds is present as carrier crystals: malic acid, tartaric acid, monosodium citrate.

12. An effervescent tablet as claimed in any of the preceding claims, wherein the first layer contains malic acid, unless the carrier crystals consist exclusively of malic acid.

13. An effervescent tablet as claimed in any of the preceding claims, having a first layer of gluconic acid delta-lactone, wherein the first layer is covered with at least one - preferably with at least two - of the following acids or alkali metal salts thereof: citric acid, malic acid, tartaric acid.

14. An effervescent tablet as claimed in any of the preceding claims, wherein the second layer is formed from a proportion of the total amount of the gas-forming component, at least partially in the form of the monosalt of the acid forming the carrier crystals and/or the first layer.

15. An effervescent tablet as claimed in any of the preceding claims, having an alkali-sensitive substance, such as, for example, acetylcysteine, captopril or minoxidil, wherein the layer applied to the carrier crystals is covered with the gas-forming components by means of an acid, preferably citric or fumaric acid.

16. An effervescent tablet as claimed in any of the preceding claims, which contains - based on 100 parts by weight of the effervescent system - at least 50, preferably at least 100, parts by weight of the pharmaceutical active substance, such as, for example, sucralfate or paracetamol, the active substance preferably being incorporated in the layer containing the gas-forming components, and wherein the carrier crystals coated in this manner are present as a mixture with gluconic acid delta-lactone and/or - in particular anhydrous - sodium carbonate, the mixture being in the form of granules.

17. A process for the preparation of an effervescent system comprising at least one solid, edible, organic acid, at least one component which forms a gas by reaction with the acid with salt formation and at least one salt formed from the acid and the gas-forming component, the surface of the crystals of the one or more acids which are present in a bed being, wet with water or with the solution of a component intended for the effervescent system and being at least partially reacted and being at most partially dried, and a further component present in finely divided form then being anchored on the surface and - optionally after partial reaction - the material being dried, wherein another acid and then at least a part of the gas-forming component are anchored to the surface of the wet acid crystals.

18. An effervescent tablet as claimed in any of the preceding claims, which contains - preferably 0.1 to 0.6 mg - of dimeticon.

**Revendications**

1. Un comprimé effervescent, contenant au moins un principe actif pharmaceutique et un système effervescent constitué d'au moins un acide solide, organique comestible se présentant sous forme solide, d'au moins un carbonate alcalin ou d'un bicarbonate utilisé comme composant permettant la libération de gaz, et d'au moins un sel alcalin de l'acide, où l'on dépose au moins deux couches sur des cristaux de support constitués au moins par l'acide en question, <u>caractérisé en ce que</u>, la première couche contient au moins un autre acide solide, organique comestible se présentant sous forme solide, et/ou le sel alcalin (de préférence acide) de cet autre acide, et en ce qu'une autre couche contient par contre au moins un sel alcalin (de préférence acide) d'au moins un des deux acides.

2. Un comprimé effervescent d'après la revendication 1, caractérisé en ce que, sur la quantité totale du ou des acides mis en jeu ainsi que sur les composants permettant la libération de gaz, il y a de 10 à 40 pour-cent en poids, en particulier de 10 à 20 pour-cent en poids, sous forme de sels, de préférence sous forme de sels acides.

3. Un comprimé effervescent d'après la revendication 1 ou 2, en particulier pour une substance active spécialement sensible à l'humidité comme, par exemple, l'Ambroxol, la Ranitidine ou la Cimétidine, caractérisé en ce que, au moins une fraction du sel et/ou du composant permettant la libération de gaz est, pour l'essentiel, présent sous forme cristalline non hydratée, où un éventuel composant permettant la libération de gaz, sous forme cristalline non hydratée, constitue de préférence la dernière couche ou couche extérieure des cristaux du support.

4. Un comprimé effervescent d'après l'une des revendications précédentes, caractérisé en ce que, le système effervescent présente un pouvoir de liaison acide d'au maximum 5 meq.

5. Un comprimé effervescent d'après l'une des revendications précédentes, caractérisé en ce qu'il contient de 0,1 à 0,6 mg de Diméticon, lequel Diméticon est déposé sur un matériau consommable neutre ou sur un support, comme, par exemple, le mannitol, le sorbitol, le lactose, la maltodextrine ou l'Aérosil.

6. Un comprimé effervescent d'après l'une des revendications précédentes, caractérisé en ce qu'il contient de quinze à trente parties en poids de matériaux consommables ou de matériaux de remplissage comme, par exemple, le manitol, le lactose ou le sorbitol, sur les 100 parties en poids du système effervescent total.

7. Comprimé effervescent d'après l'une des revendications précédentes, caractérisé en ce qu'il contient au moins deux acides organiques comestibles ayant différentes valeurs de pK, en particulier l'acide citrique et l'acide malique, de préférence dans un rapport allant de 20:1 à 3:1, en particulier, de 12:1 à 8:1.

8. Comprimé effervescent d'après l'une des revendications précédentes, caractérisé en ce que les composants permettant la libération de gaz sont présents en quantités inférieures au rapport stoechiométrique (de préférence, en une quantité ne permettant que la formation du sel simple).

9. Comprimé effervescent d'après l'une des revendications précédentes, caractérisé en ce que d'environ 5 à environ 20 pour-cent en poids, plus particulièrement, de 10 pour-cent à 15 pour-cent en poids par rapport à la quantité totale des composants permettant la libération de gaz sont présents à l'intérieur d'une deuxième ou d'une troisième couche ayant complètement réagi pour donner le sel simple, alors que le reste qui se trouve à l'intérieur de la couche qui vient par-dessus n'est fixé au cristal de support que par une réaction de liaison.

10. Comprimé effervescent d'après l'une des revendications précédentes, caractérisé en ce qu'un acide, d'abord cité respectivement sur la liste incluant l'acide citrique, l'acide malique, l'acide tartrique et l'acide adipique, est présent au sein du noyau porteur sur la surface duquel, au moins un des acides suivants ou de leurs sels, est lié (de préférence par diffusion à l'intérieur du réseau cristallin du noyau porteur) et est recouvert au moins partiellement par au moins une partie du constituant permettant le dégagement de gaz, lors de la formation d'une couche intercalaire constituée par le sel.

**11.** Comprimé effervescent d'après l'une des revendications précédentes, caractérisé en ce qu'il contient un mélange d' acide citrique, considéré comme cristal de support, avec au moins un des composés suivants : l'acide malique, l'acide tartrique ou le citrate monosodique.

**12.** Comprimé effervescent d'après l'une des revendications précédentes, caractérisé en ce que la première couche (à partir du moment où les cristaux de support ne sont pas exclusivement constitués d'acide malique) contient de l'acide malique.

**13.** Comprimé effervescent d'après l'une des revendications précédentes, avec une première couche constituée par le delta-lactone de l'acide gluconique, caractérisé en ce que la première couche est recouverte d'au moins un, de préférence, d'au moins deux des acides suivants ou de leurs sels alcalins : l'acide citrique, l'acide malique ou l'acide tartrique.

**14.** Comprimé effervescent d'après l'une des revendications précédentes, caractérisé en ce que la seconde couche est constituée par une quantité partielle de la quantité totale du composant permettant le dégagement gazeux, au moins en partie en tant que sel simple de l'acide qui constitue les cristaux du support et/ou la première couche.

**15.** Comprimé effervescent d'après l'une des revendications précédentes, avec une substance active sensible aux alcalis comme, par exemple, l'acétylcystéine, le Captopril ou le Minoxidil, caractérisé en ce que la couche déposée sur les cristaux du support est recouverte par les composants permettant le dégagement gazeux, à l'aide d'un acide, de préférence, de l'acide citrique ou de l'acide fumarique.

**16.** Comprimé effervescent d'après l'une des revendications précédentes, caractérisé en ce qu'il contient (par rapport à 100 parties en poids du système effervescent) au moins 50, de préférence au moins 100 parties en poids de la substance pharmaceutiquement active comme, par exemple, le Sucralfate ou le Paracétamol, où la substace active est encapsulée, de préférence, à l'intérieur de la couche qui contient les composants qui permettent le dégagement gazeux, et en ce que les cristaux porteurs ainsi recouverts existent à l'état granulé, mélangés avec le delta-lactone de l'acide gluconique et/ou du carbonate de sodium (plus particulièrement sous forme cristalline anhydre).

**17.** Procédé pour fabriquer un système effervescent constitué d'au moins un acide organique comestible, à l'état solide, d'au moins un composant permettant le dégagement gazeux lors de la formation du sel par réaction avec l'acide et d'au moins un sel formé à partir de l'acide et du composant permettant le dégagement gazeux, où la surface des cristaux qui se trouvent dans un déversoir et qui sont constitués de l'un au moins des acides, est arrosée avec de l'eau ou avec la solution des composants prévus pour le système effervescent, et on fait réagir au moins en partie la surface des cristaux, de même, on-sèche les cristaux, au mieux, en partie, ce sur quoi on fixe à la surface un autre composant présent en grains serrés et, le cas échéant, après une réaction partielle, on fait sécher la masse du produit, <u>caractérisé en ce que</u>, on a fixé sur la surface arrosée des cristaux d'acide un autre acide, et immédiatement après, au moins une partie du composant qui permet le dégagement gazeux.

**18.** Comprimé effervescent d'après l'une des revendications précédentes, caractérisé en ce qu'il contient de préférence de 0,1 à 0,6 mg de Diméticon.